# FASCICULE DE BREVET EUROPEEN

(11) **EP 3 593 353 B1**
(45) Date de publication et mention de la délivrance du brevet: **10.04.2024**
(21) Numéro de dépôt: 18703598.5
(22) Date de dépôt: 08.02.2018
(51) Int. Cl.: G16H 20/13, A61J 1/03

(54) **PROCÉDÉ ET DISPOSITIF DE PRÉPARATION D'UN PILULIER**
VERFAHREN UND VORRICHTUNG ZUM HERSTELLEN EINER TABLETTENBOX
METHOD AND DEVICE FOR PRODUCING A PILL BOX

(30) Priorité: 10.03.2017 FR 1751996
(43) Date de publication de la demande: 15.01.2020
(73) Titulaire: OREUS, 31380 Montastruc-la-Conseillère (FR)
(72) Inventeur: FOUBET, Michel, 31240 Saint-Jean (FR); FOUBET, Grégory, 31000 Toulouse (FR); FOUBET, Olivier, 82710 Bressols (FR)
(74) Mandataire: Bandpay & Greuter
(86) Numéro de dépôt international: PCT/EP2018/053200
(87) Numéro de publication internationale: WO 2018/162168

(56) Documents cités:
- EP-A1- 3 025 697
- EP-A2- 1 267 155
- WO-A1-2015/113167
- WO-A2-02/25568
- DE-A1-102011 086 575
- Pharmacy U: "McKesson Canada - BlistAssist", Youtube, 16 November 2016 (2016-11-16), page 1 pp., XP054981109, Retrieved from the Internet: URL:https://www.youtube.com/watch?v=f4cdnh i6Vx0 [retrieved on 2020-11-16]

## Description

L'invention concerne un procédé de préparation d'un pilulier comprenant une pluralité de logements de réception d'au moins un médicament, dit pilule, sous une forme galénique solide divisée (c'est-à-dire à l'état solide et divisé, par exemple choisie dans le groupe formé des comprimés, des cachets, des gélules, des capsules, des pilules, des pastilles, des dragées ; par opposition à un état solide massique ou compact tel que les pâtes, les pommades...) -au moins non intégralement pulvérulente voire non pulvérulente-, dans lequel on place au moins une pilule dans au moins un logement du pilulier selon des données de remplissage d'une prescription médicale pour un traitement thérapeutique d'un patient. L'invention concerne également un dispositif de préparation d'un pilulier.

Dans le cadre d'un traitement thérapeutique d'un patient, des médicaments, dits pilules, sous une forme galénique solide divisée peuvent être distribués par un personnel soignant au patient selon une prescription médicale. Cette prescription médicale indique notamment la posologie, la fréquence de prise de médicaments ainsi que la durée du traitement.

Afin de faciliter la distribution des médicaments au patient, les pilules à distribuer sont généralement préparées en amont de la distribution en étant rassemblées dans un emballage, dit pilulier, pouvant comprendre une pluralité de logements de réception de pilules (par exemple tel que décrit par FR 2 913 333). Cette étape de préparation est généralement effectuée manuellement par un personnel soignant.

Lors de cette étape de préparation, au moins une pilule est placée dans au moins un logement du pilulier selon une prescription médicale d'un unique patient. Plus particulièrement, le pilulier peut être un pilulier journalier ou un pilulier hebdomadaire par exemple.

Plus particulièrement, dans certains piluliers, toutes les pilules placées dans un même logement sont identiques, c'est-à-dire qu'elles présentent une même forme galénique, un même dosage, une même substance active, une même taille...

L'étape de préparation est méticuleuse et nécessite une concentration et une précision gestuelle du personnel soignant lors du placement manuel des pilules dans les logements du pilulier.

En particulier, l'étape de préparation est d'autant plus méticuleuse lorsque les logements d'un pilulier sont petits et proches les uns par rapport aux autres.

Lorsque les conditions de concentration et de précision gestuelle du personnel soignant ne sont pas réunies, il est possible que des pilules ne soient pas placées dans les bons logements. En effet, il est possible qu'en cas de fatigue et/ou d'inattention du personnel soignant, ce dernier puisse placer malencontreusement une pilule dans un mauvais logement ou se tromper de pilule à placer. En outre, il est possible qu'une pilule rebondisse, lors de son placement, sur une paroi d'un logement ou sur une autre pilule, dans un mauvais logement.

Or, un mauvais placement des pilules dans le pilulier peut entraîner des erreurs d'administration, et donc des conséquences néfastes chez le patient après distribution. Par exemple, le traitement peut perdre de son efficacité thérapeutique voire conduire au développement d'effets secondaires préjudiciables au patient.

DE 102011086575 décrit un procédé de remplissage dans lequel on place un pilulier présentant une pluralité de logements de réception de pilules sur un écran. On place ensuite un médicament dans un logement d'un pilulier à préparer à partir de données de remplissage associées à un patient puis on réalise une acquisition d'une image du pilulier. Cette image est ensuite affichée sur une zone de l'écran sur laquelle le pilulier n'est pas placé puis est stockée dans une base de données. Ces étapes sont répétées jusqu'à ce que l'ensemble des logements du pilulier soit rempli.

Le procédé décrit par DE 102011086575 permet d'effectuer un suivi du remplissage du pilulier en archivant les images dans une base de données. Néanmoins, il ne permet pas d'éviter les inconvénients susmentionnés.

L'invention vise à pallier ces inconvénients.

Elle vise en particulier à proposer un procédé de préparation d'un pilulier fiable.

L'invention vise également à proposer un tel procédé de préparation qui soit simple à mettre en oeuvre.

L'invention vise par ailleurs à proposer un dispositif de préparation d'un pilulier adapté pour mettre en oeuvre un tel procédé.

L'invention vise également à proposer un tel dispositif qui soit peu coûteux.

Pour ce faire, l'invention concerne un procédé de préparation d'un pilulier comprenant une pluralité de logements de réception d'au moins un médicament, dit pilule, sous une forme galénique solide divisée -au moins non intégralement pulvérulente voire non pulvérulente-,
procédé dans lequel on réalise :
- une étape, dite étape initiale, dans laquelle on place les logements du pilulier en regard d'un dispositif d'acquisition d'images,
- au moins deux étapes de remplissage dans lesquelles on place à chaque étape de remplissage un ensemble d'au moins une pilule dans au moins un logement du pilulier selon des données, dite données de remplissage, d'une prescription médicale pour un traitement thérapeutique d'un patient,
- au moins deux étapes d'acquisition d'image du pilulier par le dispositif d'acquisition d'images dont :
   ∘ une étape d'acquisition d'une image du pilulier après une étape de remplissage,
   ∘ une autre étape d'acquisition d'une image du pilulier après une autre étape de remplissage,
   caractérisé en ce qu'on réalise, après au moins une -notamment chaque- étape d'acquisition, dite étape d'acquisition courante, une étape, mise en oeuvre par ordinateur, de contrôle du remplissage du pilulier dans laquelle :
   ∘ une image acquise lors de ladite é
   ∘ tape d'acquisition courante est comparée à une image acquise lors d'une étape d'acquisition précédant ladite étape d'acquisition courante de façon à identifier les pilules placées entre cette étape d'acquisition précédant ladite étape d'acquisition courante et l'étape d'acquisition courante,
   ∘ puis une conformité du remplissage est évaluée à partir d'une comparaison entre les pilules identifiées et les données de remplissage.

L'invention s'étend à un dispositif de préparation d'un pilulier comprenant une pluralité de logements de réception d'au moins un médicament, dit pilule, sous une forme galénique solide divisée -au moins non intégralement pulvérulente voire non pulvérulente-,
dispositif de préparation comprenant un dispositif d'acquisition d'images adapté pour :
   - être placé en regard des logements du pilulier lors d'au moins deux étapes de remplissage dans lesquelles on place à chaque étape de remplissage un ensemble d'au moins une pilule dans au moins un logement du pilulier selon des données, dite données de remplissage, d'une prescription médicale pour un traitement thérapeutique d'un patient,
   - effectuer au moins deux acquisitions d'image du pilulier par le dispositif d'acquisition d'images dont :
      ∘ une acquisition d'une image du pilulier après une étape de remplissage,
      ∘ une autre acquisition d'une image du pilulier après une autre étape de remplissage,
caractérisé en ce qu'il comprend un dispositif de traitement de données comprenant des moyens de mettre en oeuvre, après au moins une -notamment chaque- étape d'acquisition, dite étape d'acquisition courante, une étape de contrôle du remplissage du pilulier dans laquelle :
   ∘ une image acquise lors de ladite étape d'acquisition courante est comparée à une image acquise lors d'une étape d'acquisition précédant ladite étape d'acquisition courante de façon à identifier les pilules placées entre cette étape d'acquisition précédant ladite étape d'acquisition courante et l'étape d'acquisition courante,
   ∘ puis une conformité du remplissage est évaluée à partir d'une comparaison entre les pilules identifiées et les données de remplissage.

L'invention s'étend également à un programme d'ordinateur comprenant des instructions de code de programme pour l'exécution des étapes d'un procédé selon l'invention lorsque ledit programme est exécuté sur un ordinateur.

En particulier, le programme d'ordinateur comprend des instructions de code de programme qui, lorsque le programme est exécuté par un ordinateur, conduisent celui-ci à mettre en oeuvre une étape de contrôle d'un remplissage d'un pilulier comprenant une pluralité de logements de réception d'au moins un médicament, dit pilule, sous une forme galénique solide divisée, dans laquelle :
∘ une image comprenant ledit pilulier comprenant un ensemble, dit premier ensemble, d'au moins une pilule placée dans au moins un logement du pilulier est comparée à une image comprenant ledit pilulier comprenant ledit premier ensemble d'au moins une pilule et un ensemble, dit deuxième ensemble, d'au moins une pilule placée dans au moins un logement du pilulier de façon à identifier chaque pilule dudit deuxième ensemble,
∘ puis une conformité du remplissage est évaluée à partir d'une comparaison entre les pilules identifiées et des données, dites de données de remplissage.

L'invention s'étend également à un support d'enregistrement lisible par ordinateur comprenant des instructions de code de programme pour l'exécution des étapes d'un procédé selon l'invention lorsqu'elles sont exécutées sur un ordinateur.

En particulier, le support d'enregistrement lisible par ordinateur comprend des instructions de code de programme qui, lorsqu'elles sont exécutées par un ordinateur, conduisent celui-ci à mettre en oeuvre une étape de contrôle d'un remplissage d'un pilulier comprenant une pluralité de logements de réception d'au moins un médicament, dit pilule, sous une forme galénique solide divisée, dans laquelle :
∘ une image comprenant ledit pilulier comprenant un ensemble, dit premier ensemble, d'au moins une pilule placée dans au moins un logement du pilulier est comparée à une image comprenant ledit pilulier comprenant ledit premier ensemble d'au moins une pilule et un ensemble, dit deuxième ensemble, d'au moins une pilule placée dans au moins un logement du pilulier de façon à identifier chaque pilule dudit deuxième ensemble,
∘ puis une conformité du remplissage est évaluée à partir d'une comparaison entre les pilules identifiées et des données, dites de données de remplissage.

Plus particulièrement, le pilulier à remplir peut par exemple être un pilulier à usage unique tel que décrit par FR 2 913 333. En particulier, le pilulier peut par exemple être utilisé pour une prescription médicale journalière ou hebdomadaire.

Les médicaments à placer lors de la préparation du pilulier peuvent être choisis dans le groupe formé notamment de comprimés, des cachets, des gélules, des capsules, des pilules, des pastilles, des dragées.

Par ailleurs, de préférence, le dispositif de traitement de données est un ordinateur, c'est-à-dire un système informatique, comprenant au moins un processeur capable de réaliser au moins l'étape de contrôle d'un procédé selon l'invention. Le dispositif de traitement de données pourrait également être un microprocesseur, un serveur...

En particulier, le dispositif de traitement de données comprend des ports d'entrée/sortie. En outre, le dispositif de traitement de données comprend également une mémoire adaptée pour stocker des données numériques.

Le dispositif d'acquisition d'images peut être un appareil photographique, une caméra, un scanner, notamment adapté pour être utilisé comme périphérique dudit dispositif de traitement de données et branché audit dispositif de traitement de données.

Telle qu'elle est utilisée dans le présent texte, l'expression «support d'enregistrement lisible par ordinateur » peut se référer à tout dispositif qui peut contenir, mémoriser, communiquer, propager ou transporter un programme pour son utilisation par ou en connexion avec un système informatique, un terminal, un appareil ou un dispositif d'exécution d'instructions de code de programme. Un tel support d'enregistrement lisible par ordinateur peut être, à titre d'exemple non limitatif, un terminal, un dispositif, un appareil, un système ou un milieu de propagation électronique, magnétique, optique, électromagnétique, infrarouge ou semi-conducteur. Certains exemples spécifiques non exhaustifs d'un support lisible par ordinateur peuvent être les suivants : un terminal informatique, une connexion électrique ayant un ou plusieurs conducteurs, une mémoire de masse (disque dur, clé USB...), une disquette, une mémoire à accès aléatoire (RAM), une mémoire à lecture seule (ROM), une mémoire à lecture seule effaçable par programmation (EPROM ou mémoire flash), une fibre optique, une mémoire à lecture optique (disque compact à lecture seule ou réinscriptible).

Dans certains modes de réalisation avantageux et selon l'invention, lors de ladite étape de contrôle du remplissage du pilulier :
∘ l'image acquise lors de ladite étape d'acquisition courante est comparée à une image acquise lors de l'étape d'acquisition qui précède chronologiquement ladite étape d'acquisition courante de façon à identifier les pilules placées entre cette étape d'acquisition qui précède chronologiquement ladite étape d'acquisition courante et l'étape d'acquisition courante,
∘ puis une conformité du remplissage est évaluée à partir d'une comparaison entre les pilules identifiées et les données de remplissage.

De préférence, on réalise une étape d'acquisition après chaque étape de remplissage.

En outre, on réalise une étape de contrôle du remplissage du pilulier après chaque étape d'acquisition.

Dans de tels modes de réalisation, un replacement des pilules mal placées, identifiées suite à une évaluation de non-conformité du remplissage, est simplifiée.

Néanmoins, dans d'autres modes de réalisation, il est possible de réaliser les étapes d'acquisition d'images qu'après un certains nombres d'étapes de remplissage entre chacune de ces étapes d'acquisition d'image. On réalise alors une étape de contrôle du remplissage après chaque étape d'acquisition d'image en comparant l'image acquise lors de cette étape d'acquisition d'image à celle acquise lors de la précédente étape d'acquisition.

Dans certains autres modes de réalisation, on réalise une étape d'acquisition d'une image après chaque étape de remplissage et on ne réalise une étape de contrôle du remplissage qu'après un certain nombre d'étapes d'acquisition d'image en comparant l'image, dite dernière image, acquise lors d'une de ces étapes d'acquisition d'image à celle acquise lors d'une de ces étapes d'acquisition et qui précède l'étape d'acquisition de ladite dernière image, plusieurs étapes d'acquisition ayant pu avoir lieu entre ces deux étapes d'acquisition.

Dans certains autres modes de réalisation, on ne réalise les étapes d'acquisition d'images qu'après un certains nombres d'étapes de remplissage entre chacune de ces étapes d'acquisition d'image et on ne réalise une étape de contrôle du remplissage qu'après un certain nombre d'étapes d'acquisition d'image en comparant l'image, dite dernière image, acquise lors d'une de ces étapes d'acquisition d'image à celle acquise lors d'une de ces étapes d'acquisition et qui précède l'étape d'acquisition de ladite dernière image, plusieurs étapes d'acquisition ayant pu avoir lieu entre ces deux étapes d'acquisition.

Dans certains modes de réalisation avantageux et selon l'invention, on place une pluralité de pilules dans au moins un logement du pilulier, toutes les pilules placées dans un même logement étant identiques.

Ainsi, à la fin d'un procédé de préparation selon l'invention, l'ensemble de pilules comprises dans chaque logement du pilulier comprenant une pluralité de pilules comprend uniquement des pilules identiques (même forme galénique, même dosage, même substance active, même taille...).

De préférence, le pilulier comprend une pluralité de portions comprenant chacune au moins un logement -notamment une pluralité de logements- chaque portion étant destinée à recevoir une prise unitaire de pilules, c'est-à-dire un ensemble de pilules destinées à être administrées sensiblement en une seule prise à un patient à un moment donné, ledit ensemble de pilules étant conforme à la posologie d'une prescription médicale destinée au traitement d'au moins une pathologie affectant ce patient. Les différents logements de chaque portion sont séparés les uns des autres de façon à isoler les uns des autres les différents médicaments d'une même prise unitaire de pilules (comme décrit par FR2913333).

De préférence, lesdites portions sont des portions détachables reliées entre elles par des liaisons sécables de façon à pouvoir détacher une portion pour chaque prise unitaire de pilules.

Pour chaque étape de remplissage, les données de remplissage comprennent des indications sur le type de pilule à placer (notamment le nom du médicament, le dosage voire également la couleur et la taille de la pilule), sur le nombre des pilules à placer dans le pilulier et sur les logements (position, numéro) dans lesquels elles doivent être placées. Ces données de remplissage sont préenregistrées dans une mémoire dudit dispositif de traitement de données et associées à un identifiant d'un patient.

En particulier, lors de l'étape initiale, on indique -notamment un personnel soignant indique-, audit dispositif de traitement de données l'identifiant du patient pour lequel le pilulier doit être préparé afin que ledit dispositif de traitement de données puisse récupérer en mémoire les données de remplissage à utiliser lors du procédé de préparation du pilulier.

Plus particulièrement, avantageusement et selon l'invention, à chaque étape de remplissage, une indication, dite indication de remplissage, relative aux données de remplissage pour cette étape de remplissage est affichée sur un écran, dit écran d'aide au remplissage. Un tel écran d'aide au remplissage est relié audit dispositif de traitement de données.

En outre, dans certains modes de réalisation avantageux et selon l'invention, on dispose l'écran d'aide au remplissage en regard des logements du pilulier et à l'opposé du dispositif d'acquisition d'images par rapport au pilulier.

Plus particulièrement, l'écran d'aide au remplissage est placé en-dessous des logements, c'est-à-dire à l'opposé d'ouvertures d'accès aux logements du pilulier.

Un tel écran d'aide au remplissage est alors adapté pour indiquer les logements dans lesquels au moins une pilule doit être placée en éclairant selon un code couleur prédéterminé des régions de l'écran placées en dessous de ces logements. En outre, le nombre de pilules à placer dans chacun de ces logements peut également être indiqué sur ces régions de l'écran.

Un tel écran d'aide au remplissage permet de faciliter le remplissage du pilulier pouvant être effectué notamment par un personnel soignant.

En particulier, dans certains modes de réalisation avantageux et selon l'invention, dans au moins une étape de remplissage on place une pluralité de pilules dans au moins un logement du pilulier, les pilules de ladite pluralité de pilules placées dans le pilulier lors d'une même étape de remplissage étant identiques.

Ainsi, on manipule -notamment un personnel soignant manipule- uniquement des pilules identiques à chaque étape de remplissage. Cela permet non seulement de réduire le risque d'erreur de placement des pilules mais également de réduire le temps de préparation du pilulier.

Par ailleurs, de préférence, on fixe le pilulier à un support de fixation agencé pour que les logements du pilulier soit en regard du dispositif d'acquisition d'images.

La fixation du pilulier permet de garder le pilulier dans une même position par rapport au dispositif d'acquisition d'images qui lui-même est fixe par rapport au support de fixation. Ainsi, l'étape de contrôle est facilitée.

Par ailleurs, dans certains modes de réalisation avantageux et selon l'invention, l'étape initiale comprend une étape d'acquisition d'une image, dite image initiale, du pilulier par le dispositif d'acquisition d'images après avoir placé le pilulier en regard du dispositif d'acquisition d'images.

De préférence, lors de cette étape initiale, le pilulier est vide de médicament.

Néanmoins, dans d'autres modes de réalisation, notamment lorsqu'un même type de pilulier est utilisé pour plusieurs procédés de préparation de pilulier, l'image initiale du pilulier est enregistrée en mémoire après une première acquisition de cette image initiale de façon à pouvoir être réutilisée pour l'ensemble des procédés de préparation de pilulier et éviter l'acquisition d'une nouvelle image initiale pour chacun de ces procédés.

L'image acquise lors de la première étape de remplissage suivant l'étape initiale est comparée à l'image initiale lors de l'étape de contrôle succédant cette première étape de remplissage.

Dans certains modes de réalisation, lorsqu'on termine une étape de remplissage, on indique audit dispositif de traitement de données par un périphérique d'entrée branché audit dispositif de traitement de données qu'on a terminé l'étape de remplissage pour pouvoir passer à l'étape d'acquisition puis à l'étape de contrôle.

En variante, une balance peut être placée sous le pilulier de façon à détecter la fin du remplissage d'une étape de remplissage en fonction de la masse du pilulier et d'une masse des médicaments indiquée dans les données de remplissage de façon à passer automatiquement à l'étape d'acquisition puis à l'étape de contrôle.

Lors de chaque étape de comparaison, on compare en particulier des données numériques, notamment des données colorimétriques, représentatives de ladite image acquise lors de ladite étape d'acquisition courante à des données numériques, notamment des données colorimétriques, représentatives de ladite image acquise lors d'une étape d'acquisition précédant ladite étape d'acquisition courante. Cette comparaison permet d'identifier, dans des données numériques (notamment des données colorimétriques) produites lors de cette comparaison, les données numériques représentatives des pilules placées entre ladite étape d'acquisition précédant ladite étape d'acquisition courante et l'étape d'acquisition courante.

En particulier, afin de comparer ladite image acquise lors de ladite étape d'acquisition courante à ladite image acquise lors d'une étape d'acquisition précédant ladite étape d'acquisition courante, il est possible d'effectuer une différence de ces deux images (notamment une différence des données colorimétriques de ces deux images) afin d'obtenir une image, dite image à analyser, avec laquelle les pilules placées lors de la précédente étape de remplissage peuvent être identifiées facilement.

En particulier, à chaque étape de contrôle la position desdites pilules identifiées est déterminée à partir de moyens de traitement et d'analyse d'image et est comparée à une position indiquée par les données de remplissage de l'étape de remplissage précédant cette étape de contrôle.

Par exemple, la position des pilules identifiées peut être déterminée à partir des données colorimétriques de ladite image à analyser. En outre, il est également possible de déterminer la position des pilules identifiées par détection des contours des pilules identifiées (notamment à partir d'une image noir et blanc issue de ladite image à analyser ou encore à partir de l'application d'un filtre Sobel sur cette image à analyser).

En outre, à chaque étape de contrôle, une quantité desdites pilules identifiées dans chaque logement est déterminée à partir de moyens de traitement et d'analyse d'images et comparée à une quantité indiquée par les données de remplissage de l'étape de remplissage précédant cette étape de contrôle.

En particulier, la quantité de pilules dans chaque logement peut être déterminée à partir de la position des pilules et des contours desdites pilules identifiées.

Dans certains modes de réalisation avantageux et selon l'invention, à chaque étape de contrôle, l'image acquise lors de ladite étape d'acquisition courante est comparée à l'image acquise lors de l'étape d'acquisition précédant ladite étape d'acquisition courante uniquement sur certaines zones, dites zones d'intérêt, correspondant à la position des logements du pilulier dont les coordonnés spatiales ont été enregistrées.

De plus, les moyens de traitement et d'analyse analysent uniquement l'image à analyser au niveau de ces zones d'intérêt.

En particulier, les coordonnés spatiales sont enregistrées en mémoire dudit dispositif de traitement de données à partir d'une image d'un pilulier acquise lors d'une étape de calibration dans laquelle ce pilulier est placé, notamment fixé, en regard du dispositif d'acquisition d'images.

Après avoir comparé ladite image acquise lors de ladite étape d'acquisition courante à ladite image acquise lors d'une étape d'acquisition précédant ladite étape d'acquisition courante on évalue la conformité du remplissage.

En particulier, la conformité du remplissage est évaluée à partir d'une comparaison entre des données numériques relatives aux pilules identifiées (notamment la position, la quantité et le type des pilules identifiées) et les données de remplissage.

L'évaluation de la conformité du remplissage permet de déterminer si les pilules placées lors de l'étape de remplissage ont été placées dans les logements indiqués par les données de remplissage. Elle permet également de déterminer si le nombre de pilules placées dans les logements correspond à celui indiqué par les données de remplissage. En outre, elle permet également de déterminer si des logements non indiqués par les données de remplissage comprennent des pilules et aussi si des pilules non indiquées par les données de remplissage sont présentes dans les logements de remplissage indiqués par les données de remplissage.

Après avoir évalué la conformité du remplissage, ledit dispositif de traitement de données est programmé pour signaler de la conformité ou non du remplissage par rapport aux données de remplissage.

En particulier, de préférence, à chaque étape de contrôle, une indication de conformité du remplissage du pilulier est affichée sur au moins un écran, dit écran de contrôle, après que la conformité du remplissage ait été évaluée.

En variante ou en combinaison, l'indication de conformité du remplissage du pilulier peut être un signal sonore.

Dans un mode de réalisation préférentiel et selon l'invention, à chaque étape de contrôle, une indication de conformité du remplissage de chacun des logements du pilulier est affichée sur au moins un écran, dit écran de contrôle, après que la conformité du remplissage ait été évaluée.

Plus particulièrement, cet écran de contrôle peut être l'écran d'aide au remplissage également (et peut donc être placé sous les logements du pilulier).

En outre, l'écran de contrôle affichant l'indication de conformité du remplissage de chacun des logements du pilulier peut être le même que l'écran de contrôle affichant l'indication de conformité du remplissage du pilulier.

Dans le cas où le remplissage est évalué comme non conforme, le passage à une prochaine étape de remplissage est empêché jusqu'à ce que les pilules soient placées conformément aux données de remplissage.

En particulier, les logements non conformes aux données de remplissage sont indiqués sur l'écran de contrôle pour qu'on puisse identifier rapidement -notamment pour qu'un personnel soignant puisse identifier rapidement- les pilules qu'on doit déplacer pour être conforme aux données de remplissage.

En particulier, les régions de l'écran de contrôle placées en regard des logements non conformes aux données de remplissage peuvent être colorées selon un code couleur prédéterminé (notamment une couleur rouge) et le nombre de pilules à placer dans ces logements peut y être indiqué également.

Les logements conformes aux données de remplissage peuvent également être indiqués sur l'écran de contrôle.

En particulier, les régions de l'écran de contrôle placées en regard des logements conformes aux données de remplissage peuvent être colorées selon un code couleur prédéterminé (par exemple une couleur verte).

Lorsque le personnel soignant a fini de replacer les pilules selon les données de remplissage, on réalise une nouvelle étape d'acquisition d'une image du pilulier puis une nouvelle étape de contrôle de cette image.

Dans le cas où le remplissage est évalué comme conforme, le passage à une prochaine étape de remplissage est autorisé.

Les logements conformes aux données de remplissage peuvent également être indiqués sur l'écran de contrôle.

En particulier, les régions de l'écran de contrôle placées en regard des logements peuvent être colorées selon un code couleur prédéterminé (par exemple une couleur verte).

Lorsque l'ensemble des étapes de remplissage du procédé de préparation a été effectué et que les logements du pilulier sont conformes à l'ensemble des données de remplissage, les logements sont recouverts d'un opercule pour sceller le pilulier.

Un procédé selon l'invention permet d'évaluer la conformité du remplissage après chaque étape de remplissage.

Un procédé selon l'invention permet donc de déterminer après chaque étape de remplissage si une erreur dans le remplissage du pilulier est survenue.

Un procédé selon l'invention permet donc d'assister un personnel soignant lors de la préparation d'un pilulier en contrôlant la conformité du remplissage des logements du pilulier.

En outre, un procédé selon l'invention permet, en contrôlant la conformité du remplissage après chaque étape de remplissage, de faciliter la correction du remplissage (si celui-ci est non conforme aux données de remplissage) par rapport à un procédé de préparation d'un pilulier dans lequel un unique contrôle du remplissage serait effectué lorsque toutes les pilules à placer sont placées dans les logements du pilulier.

Un procédé selon l'invention est simple et facile à mettre en oeuvre.

En outre, un dispositif de préparation d'un pilulier selon l'invention est peu coûteux.

L'invention concerne également un procédé de préparation d'un pilulier, un dispositif de préparation d'un pilulier, un programme d'ordinateur et un support d'enregistrement lisible par ordinateur caractérisés en combinaison par tout ou partie des caractéristiques mentionnées ci-dessus ou ci-après.

D'autres buts, caractéristiques et avantages de l'invention apparaîtront à la lecture de la description suivante donnée à titre non limitatif et qui se réfère aux figures annexées dans lesquelles :
- la figure 1 est un schéma d'un dispositif de préparation d'un pilulier selon un mode de réalisation de l'invention,
- la figure 2 est un schéma séquentiel d'un procédé de préparation d'un pilulier selon un mode de réalisation de l'invention,
- la figure 3 est un schéma d'un pilulier sur lequel sont représentées des zones, dites zones d'intérêt.

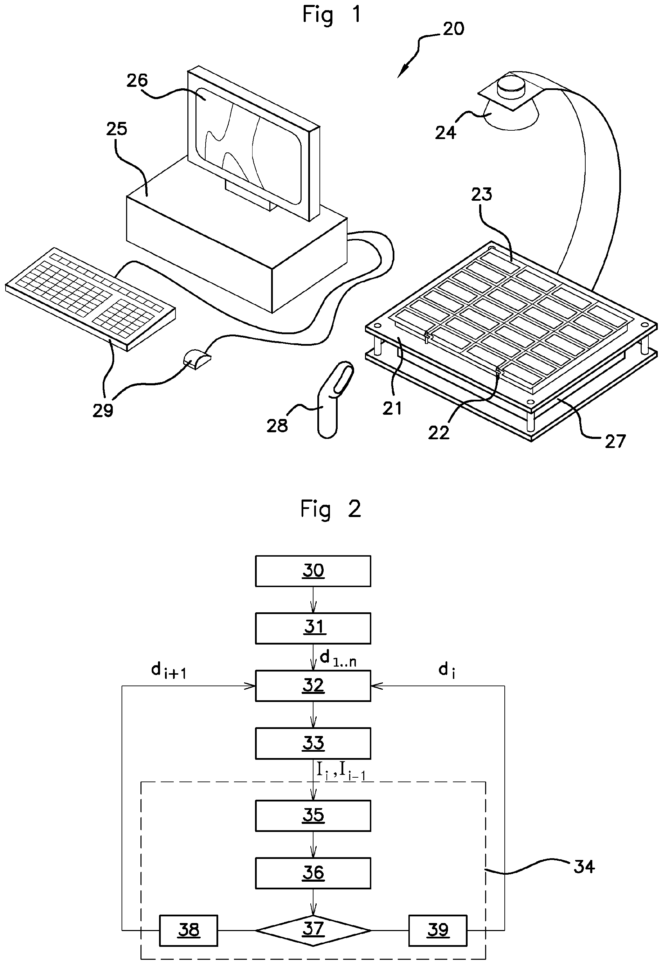

Un dispositif 20, représenté à la figure 1, de préparation d'un pilulier 23 selon l'invention comprend un support 21 de fixation de pilulier 23.

Le support 21 de fixation comprend des ergots 22 de fixation de piluliers 23 adaptés pour venir être placés en butée d'un pilulier 23 pour maintenir fixement ledit pilulier 23.

Plus particulièrement, le pilulier 23 comprend des ouvertures 42 de réception des ergots 22 de fixation sur au moins un bord du pilulier 23.

Le support 21 de fixation peut par exemple être celui décrit par FR 2 942 132.

Le pilulier 23 comprend une pluralité de logements 40 de réception d'au moins un médicament, dit pilule 41, sous une forme galénique solide divisée au moins non intégralement pulvérulente (par exemple des gélules) voire non pulvérulente (par exemple des comprimés).

De préférence, le pilulier 23 comprend une pluralité de portions comprenant chacune au moins un logement 40 -notamment une pluralité de logements 40- destiné à recevoir une prise unitaire de pilules, c'est-à-dire un ensemble de pilules destinées à être administrées sensiblement en une seule prise à un patient à un moment donné, ledit ensemble de pilules étant conforme à la posologie d'une prescription médicale destinée au traitement d'au moins une pathologie affectant ce patient.

En outre, dans chacune de ladite pluralité de portions, les logements 40 adjacents compris dans cette portion peuvent être séparés par une paroi commune, notamment une paroi amovible. En variante, les logements 40 adjacents compris dans une portion de ladite pluralité de portions peuvent être espacés les uns des autres.

De préférence, lesdites portions sont détachables et reliées entre elles par des liaisons sécables de façon à pouvoir détacher une portion pour chaque prise unitaire de pilules.

Le support 21 de fixation permet de positionner les logements 40 du pilulier 23 en regard d'un dispositif 24 d'acquisition d'images placé lui-même en regard du support 21 de fixation.

Le dispositif 24 d'acquisition d'images est donc agencé et adapté pour effectuer des acquisitions d'images d'un pilulier 23 placé en regard de ce dispositif 24 d'acquisition d'images, notamment au-dessus du pilulier 23, c'est-à-dire en regard d'ouvertures d'accès des logements 40.

Le dispositif 24 d'acquisition d'images est de préférence fixé rigidement au support 21 de fixation du pilulier.

Le dispositif 24 d'acquisition d'images peut être un appareil photographique, une caméra, un scanner 28, notamment adapté pour être utilisé comme périphérique dudit dispositif 25 de traitement de données et branché audit dispositif 25 de traitement de données.

Le dispositif 24 d'acquisition d'images est relié à un dispositif 25 de traitement de données. Ce dispositif 25 de traitement de données peut par exemple être un ordinateur.

Le dispositif 25 de traitement de données comprend en particulier des moyens de traitement et d'analyse d'images. En outre, le dispositif 25 de traitement de données comprend en particulier une mémoire.

Ledit dispositif 25 de traitement de données comprend également un programme d'ordinateur adapté pour utiliser lesdits moyens de traitement et d'analyse d'images.

En particulier ce programme d'ordinateur est enregistré sur un support d'enregistrement lisible par ordinateur. De préférence, ledit support d'enregistrement est la mémoire du dispositif 25 de traitement de données.

Ledit programme d'ordinateur est utilisé lors d'un procédé selon l'invention de préparation d'un pilulier 23, notamment lors d'étapes de remplissage du pilulier 23, d'acquisitions d'images du pilulier 23 et de contrôle du remplissage du pilulier 23.

En outre, le dispositif 25 de traitement de données est relié à un écran 26 principal sur lequel peut être affiché une interface graphique à partir dudit programme d'ordinateur.

Un procédé de préparation d'un pilulier 23, représenté selon un mode réalisation de l'invention à la figure 2, comprend une étape 30 initiale dans laquelle on place et on fixe un pilulier 23 vide comprenant une pluralité de logements 40 de réception d'au moins une pilule 41 sur ledit support 21 de fixation de sorte que les logements 40 du pilulier 23 soient en regard du dispositif 24 d'acquisition d'images.

De préférence, lors de cette étape 30 initiale, une image, dite image initiale, du pilulier 23 vide est acquise par le dispositif 24 d'acquisition d'images et transmise au dispositif 25 de traitement de données qui l'enregistre en mémoire.

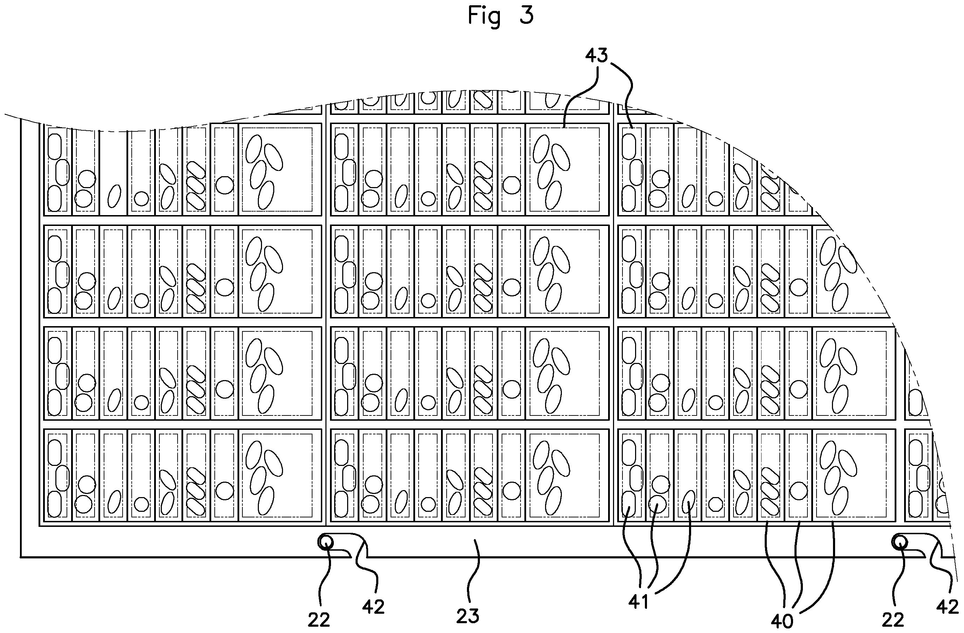

En outre des zones, dites zones 43 d'intérêt, représentées en exemple sur la figure 3, correspondant à la position des logements 40 du pilulier 23 sont définies lors d'une étape de calibration (pouvant être comprise dans l'étape 30 initiale ou la précéder) et leurs coordonnés spatiales sont enregistrées en mémoire du dispositif 25 de traitement de données.

L'acquisition d'une image du pilulier 23 vide et une définition de zones 43 d'intérêt peuvent être réalisées dès qu'un procédé de préparation d'un pilulier 23 selon l'invention est réalisé. En variante, il est possible d'effectuer une unique acquisition d'une image d'un pilulier 23 vide et une unique définition de zones 43 d'intérêt pour plusieurs procédés selon l'invention de préparation lorsque les piluliers 23 à préparer lors de ces procédés sont identiques.

Un procédé de préparation d'un pilulier 23 comprend également une étape 31 de chargement de données *d_{1..n},* dites données de remplissage, (*n* étant égal au nombre d'étapes de remplissage décrites ci-dessous du procédé de préparation du remplissage) par le dispositif 25 de traitement de données dans laquelle on sélectionne un patient dans une base de données stockée en mémoire du dispositif 25 de traitement de données selon un identifiant donné du patient, puis le dispositif 25 de traitement de données extrait les données *d*_{*1*..*n*} de remplissage stockées en mémoire du dispositif 25 de traitement et liées à un identifiant donné du patient.

Les données *d*_{*1*..*n*} de remplissage sont relatives à une prescription médicale du patient auquel elles sont liées. Les données *d_{1..n}* de remplissage comprennent donc des indications sur une posologie de la prescription et donc des indications sur le remplissage du pilulier 23 à préparer en vue de sa distribution au patient. En particulier, les données *d_{1..n}* de remplissage comprennent des indications concernant la position des pilules 41 à placer dans les logements 40 du pilulier 23, la quantité de chaque pilule 41 dans chaque logement 40 du pilulier 23 et le type de chacune de ces pilules 41.

Un procédé selon l'invention comprend au moins deux étapes 32 de remplissage du pilulier 23 après l'étape 31 de chargement des données *d_{1..n}* de remplissage.

En outre, les données *d_{1..n}* de remplissage sont divisées en données *dᵢ* de remplissage, *i* étant compris entre 1 et *n,* relatives à une étape *i* de remplissage d'un procédé de préparation d'un pilulier 23 selon l'invention.

Ainsi, à chaque étape *i* de remplissage, on place un ensemble d'au moins une pilule 41 dans au moins un logement 40 du pilulier 23 selon un arrangement indiqué par les données *dᵢ* de remplissage qui sont relatives à cette étape de remplissage.

Avant d'être placées dans les logements 40 du pilulier 23, les pilules 41 sont conditionnées dans des boîtes à pilules 41, chaque boîte comprenant un ensemble de pilules 41 identiques les unes par rapport aux autres (même forme galénique, même dosage, même substance active, même taille...).

Chaque boîte de pilules 41 présente avantageusement un code-barres.

De préférence, les pilules 41 à placer lors d'une étape de remplissage sont identiques les unes par rapport aux autres (même forme galénique, même dosage, même substance active, même taille...).

Ainsi, on manipule uniquement des pilules 41 identiques à chaque étape de remplissage. Cela permet non seulement de réduire le risque d'erreur de placement des pilules 41 mais également de réduire le temps de préparation du pilulier 23.

En outre, avant de placer les pilules 41 dans au moins un logement 40 du pilulier 23 à chaque étape de remplissage, il est préférable de lire, à l'aide d'un scanner 28 du dispositif 20 de préparation, le code-barres de la boîte comprenant les pilules 41 à placer lors de cette étape de remplissage.

La lecture du code-barres permet d'indiquer au dispositif 25 de traitement de données qui est relié au scanner 28 les pilules 41 qui vont être placées lors de l'étape de remplissage.

De préférence, à chaque étape de remplissage, chaque logement 40 dans lequel on doit placer au moins une pilule 41 lors d'une étape de remplissage est indiqué par une indication de remplissage sur un écran 27 d'aide au remplissage placé en regard des logements 40 du pilulier 23 en-dessous du pilulier 23, et donc à l'opposé du dispositif 24 d'acquisition par rapport au pilulier 23.

Plus particulièrement, des régions de l'écran 27 d'aide au remplissage placées en regard des logements 40 dans lesquels au moins une pilule 41 doit être placée sont éclairées selon un code couleur prédéterminé. En outre, pour chacune de ces régions de l'écran 27 d'aide au remplissage, un numéro peut être affiché sur cette région de façon à indiquer le nombre de pilules 41 devant être placées dans le logement 40 en regard de cette région.

Dans le mode de réalisation représenté à la figure 2, un procédé selon l'invention comprend, après chaque étape *i* de remplissage, une étape *i* d'acquisition (étape 33 sur la figure 2) d'une image du pilulier 23 par le dispositif 24 d'acquisition d'images.

En particulier, lorsqu'on termine une étape *i* de remplissage, on indique audit dispositif 25 de traitement de données par un périphérique 29 d'entrée branché audit dispositif 25 de traitement de données qu'on a terminé l'étape *i* de remplissage pour pouvoir passer à l'étape *i* d'acquisition.

En variante, une balance peut être placée sous le pilulier 23 de façon à détecter la fin du remplissage d'une étape *i* de remplissage en fonction de la masse du pilulier 23 et d'une masse des médicaments indiquée dans les données de remplissage de façon à passer automatiquement à l'étape *i* d'acquisition.

Dans le mode de réalisation représenté à la figure 2, un procédé selon l'invention comprend, après chaque étape *i* d'acquisition, une étape 34 de contrôle du remplissage du pilulier 23.

En particulier, l'étape 34 de contrôle du remplissage du pilulier 23 comprend une étape 35 de comparaison de l'image *Iᵢ* acquise lors de l'étape *i* d'acquisition par rapport à l'image *Iᵢ₋₁* acquise lors de l'étape *i-1* d'acquisition, *i* étant compris entre 1 et *n, I₀* étant l'image initiale et l'étape 0 d'acquisition étant l'étape 30 initiale.

L'étape *i-1* d'acquisition est donc l'étape d'acquisition qui précède chronologiquement l'étape *i* d'acquisition dans l'ensemble des étapes d'acquisition du procédé de préparation du pilulier 23.

Sur l'image *Iᵢ₋₁*, le pilulier 23 comprend un ensemble, dit premier ensemble, d'au moins une pilule 41, si *i* est supérieur à 1 (aucune pilule 41 pour l'image I*₀* sur laquelle le pilulier 23 est vide). Ce premier ensemble comprend les pilules 41 placées lors de l'étape *i-1* de remplissage qui précède l'étape *i-1* d'acquisition et les pilules 41 placées avant cette étape *i-1* de remplissage.

Sur l'image *Iᵢ*, le pilulier 23 comprend le premier ensemble d'au moins une pilule 41 et un deuxième ensemble de pilules 41 comprenant les pilules 41 placées lors de l'étape *i* de remplissage qui précède l'étape *i* d'acquisition

Le programme d'ordinateur est programmé pour comparer l'image *Iᵢ* par rapport à l'image *Iᵢ₋₁* de façon à identifier chaque pilule 41 dudit deuxième ensemble de pilules 41, et donc chaque pilule 41 ayant été placée dans le pilulier 23 lors de l'étape *i* de remplissage.

En particulier, lors de l'étape 35 de comparaison, le dispositif 25 de traitement de données effectue grâce audit programme d'ordinateur une différence entre l'image *Iᵢ* et l'image *Iᵢ₋₁* (notamment une différence des données colorimétriques de ces deux images) de façon à identifier chaque pilule 41 dudit deuxième ensemble de pilules 41. La différence entre l'image *Iᵢ* et l'image *Iᵢ₋₁* permet d'obtenir une image, dite image à analyser, sur laquelle seule les pilules 41 placées lors de l'étape *i* de remplissage sont présentes de sorte qu'elles peuvent être identifiées facilement lors d'une étape 36 d'analyse.

Lorsque chaque pilule 41 dudit deuxième ensemble d'au moins une pilule 41 a été identifiée, le dispositif 25 de traitement détermine grâce audit programme d'ordinateur la position de chaque pilule 41 de ce deuxième ensemble.

La position des pilules 41 est déterminée à partir de moyens, notamment d'algorithmes, de traitement et d'analyse d'images programmés dans le programme d'ordinateur.

Par exemple, la position des pilules 41 peut être déterminée à partir des données colorimétriques de l'image à analyser. En outre, il est également possible de déterminer la position des pilules 41 identifiées par identification des contours des pilules 41 présentes sur l'image à analyser (notamment à partir d'une image noir et blanc issue de l'image à analyser ou encore à partir de l'application d'un filtre Sobel sur l'image à analyser).

De plus, lorsque chaque pilule 41 dudit deuxième ensemble d'au moins une pilule 41 a été identifiée, le dispositif 25 de traitement détermine également une quantité desdites pilules 41 identifiées dans chaque logement 40 du pilulier 23 grâce audit programme d'ordinateur la position de chaque pilule 41 de ce deuxième ensemble.

Ladite quantité des pilules 41 identifiées peut également être déterminée à partir de moyens, notamment d'algorithmes, de traitement et d'analyse d'images programmés dans le programme d'ordinateur.

En particulier, la quantité de pilules 41 dans chaque logement 40 peut être déterminée à partir de la position des pilules 41 et des contours desdites pilules 41 identifiées.

En outre, le dispositif 25 de traitement peut également déterminer grâce audit programme d'ordinateur le type (notamment le nom du médicament) de chaque pilule 41 de ce deuxième ensemble.

Le type de chaque pilule 41 dudit deuxième ensemble peut être déterminé à partir de moyens, notamment d'algorithmes, de traitement et d'analyse d'images programmés dans le programme d'ordinateur.

En particulier, le type de chaque pilule 41 dudit deuxième ensemble peut être déterminé à partir d'une analyse colorimétrique de l'image à analyser.

De préférence, l'analyse de la position, de la quantité et du type de chaque pilule 41 dudit deuxième ensemble est effectué uniquement au niveau desdites zones 43 d'intérêt de façon à réduire le nombre de calculs à effectuer par le dispositif 25 de traitement de données.

La position déterminée de chaque pilule 41, la quantité déterminée de chaque pilule 41 dans chaque logement 40 et le type déterminé de chaque pilule 41 sont ensuite comparés lors d'une étape 37 à la position de chaque pilule 41, la quantité de chaque pilule 41 dans chaque logement 40 et au type de chaque pilule 41 indiqués par les données *dᵢ* de remplissage.

Plus particulièrement, le traitement et l'analyse de l'image à analyser par rapport aux données de remplissage permet de déterminer si les pilules 41 placées lors de l'étape *i* de remplissage ont été placées dans les logements 40 indiqués par les données *dᵢ* de remplissage. Ce traitement et cet analyse permet également de déterminer si le nombre de pilules 41 placées dans les logements 40 correspond à celui indiqué par les données *dᵢ* de remplissage. En outre, ils permettent également de déterminer si des logements 40 non indiqués par les données *dᵢ* de remplissage comprennent des pilules 41 et aussi si des pilules 41 non indiquées par les données *dᵢ* de remplissage sont présentes dans les logements 40 de remplissage indiqués par les données *dᵢ* de remplissage.

Après avoir évalué la conformité du remplissage effectué lors de l'étape *i* de remplissage, le programme d'ordinateur est programmé pour signaler de la conformité ou non de ce remplissage.

En particulier, l'écran 27 d'aide au remplissage est utilisé de façon à signaler la conformité ou non de remplissage lors d'une étape 38 lorsque le remplissage lors d'une étape de remplissage du pilulier 23 est évalué comme conforme et lors d'une étape 39 lorsque le remplissage lors d'une étape de remplissage du pilulier 23 est évalué comme non conforme.

Dans le cas où le remplissage effectué lors de l'étape *i* de remplissage est évalué comme non conforme, le passage à une prochaine étape *i*+*1* de remplissage est empêché (en affichant par exemple un message de non-conformité du remplissage dans une fenêtre modale sur l'écran 26 principal) jusqu'à ce que les pilules 41 soient placées conformément aux données *dᵢ* de remplissage.

En particulier, les logements 40 non conformes aux données *dᵢ* de remplissage sont indiqués lors de l'étape 39 par des indications de conformité du remplissage sur l'écran 27 d'aide au remplissage pour qu'on puisse identifier rapidement les pilules 41 qu'il doit déplacer pour être conforme aux données *dᵢ* de remplissage.

En particulier, les régions de l'écran 27 d'aide au remplissage placées en regard des logements 40 non conformes aux données *dᵢ* de remplissage peuvent être colorées selon un code couleur prédéterminé (notamment une couleur rouge) et le nombre de pilules 41 à placer dans ces logements 40 peut y être indiqué également.

Les logements 40 conformes aux données *dᵢ* de remplissage peuvent également être indiqués lors de l'étape 39 par d'autres indications de remplissage sur l'écran 27 d'aide au remplissage.

En particulier, les régions de l'écran 27 d'aide au remplissage placées en regard des logements 40 conformes aux données *dᵢ* de remplissage peuvent être colorées selon un code couleur prédéterminé (par exemple une couleur verte).

Lorsqu'on a fini de replacer les pilules 41 selon les données *dᵢ* de remplissage, on réalise une nouvelle étape *i* d'acquisition d'une image *Iᵢ* du pilulier 23 puis une nouvelle étape 34 de contrôle du remplissage dans laquelle on compare toujours l'image *Iᵢ* à l'image *Iᵢ₋₁* de l'étape *i-1* d'acquisition sur laquelle le pilulier 23 comprend uniquement ledit premier ensemble d'au moins une pilule 41.

Dans le cas où le remplissage est évalué comme conforme, le passage à une prochaine étape de remplissage selon des données *d*_{*i*+*1*} de remplissage est autorisé par le programme d'ordinateur.

Les logements 40 conformes aux données *dᵢ* de remplissage peuvent également être indiqués lors de l'étape 38 par des indications de conformité du remplissage sur l'écran 27 d'aide au remplissage.

En particulier, les régions de l'écran 27 d'aide au remplissage placées en regard des logements 40 peuvent être colorées selon un code couleur prédéterminé (par exemple une couleur verte).

Lorsque l'ensemble des étapes de remplissage du procédé de préparation a été effectué et que les logements 40 du pilulier 23 sont conformes à l'ensemble des données de remplissage, les logements 40 sont recouverts d'un opercule pour sceller le pilulier 23.

L'invention concerne donc un procédé de préparation d'un pilulier 23, un dispositif 20 de préparation d'un pilulier 23, un programme d'ordinateur et un support d'enregistrement lisible par ordinateur permettant de placer des pilules 41 dans un pilulier 23 selon une prescription médicale d'un patient de façon fiable et peu coûteuse.

Un procédé selon l'invention de préparation d'un pilulier 23 peut être réalisé par un personnel soignant.

L'invention peut faire l'objet de nombreuses variantes de réalisation par rapport aux modes de réalisation décrits ci-dessus et représentés sur les figures. En particulier, l'étape 30 initiale et les étapes 32 de remplissage peuvent être réalisées par une machine. En outre, l'étape 34 de contrôle peut ne pas être effectuée après chaque étape d'acquisition.

## Revendications

1. - Procédé de préparation d'un pilulier (23) comprenant une pluralité de logements (40) de réception d'au moins un médicament, dit pilule (41), sous une forme galénique solide divisée,
procédé dans lequel on réalise :
- une étape, dite étape (30) initiale, dans laquelle on place les logements (40) du pilulier (23) en regard d'un dispositif (24) d'acquisition d'images,
- au moins deux étapes de remplissage dans lesquelles on place à chaque étape de remplissage un ensemble d'au moins une pilule (41) dans au moins un logement (40) du pilulier (23) selon des données, dite données de remplissage, d'une prescription médicale pour un traitement thérapeutique d'un patient,
- au moins deux étapes (35) d'acquisition d'image du pilulier (23) par le dispositif (24) d'acquisition d'images dont :
o une étape d'acquisition d'une image du pilulier (23) après une étape de remplissage,
o une autre étape d'acquisition d'une image du pilulier (23) après une autre étape de remplissage,
**caractérisé en ce qu'**on réalise, après au moins une étape d'acquisition, dite étape d'acquisition courante, une étape (34), mise en oeuvre par ordinateur, de contrôle du remplissage du pilulier (23) dans laquelle :
o une image acquise lors de ladite étape d'acquisition courante est comparée à une image acquise lors d'une étape d'acquisition précédant ladite étape d'acquisition courante de façon à identifier les pilules (41) placées entre cette étape d'acquisition précédant ladite étape d'acquisition courante et l'étape d'acquisition courante,
o puis une conformité du remplissage est évaluée à partir d'une comparaison entre les pilules (41) identifiées et les données de remplissage.

2. - Procédé selon la revendication 1 **caractérisé en ce que** lors de ladite étape (34) de contrôle du remplissage du pilulier :
o l'image acquise lors de ladite étape d'acquisition courante est comparée à une image acquise lors de l'étape d'acquisition qui précède chronologiquement ladite étape d'acquisition courante de façon à identifier les pilules (41) placées entre cette étape d'acquisition qui précède chronologiquement ladite étape d'acquisition courante et l'étape d'acquisition courante,
o puis une conformité du remplissage est évaluée à partir d'une comparaison entre les pilules (41) identifiées et les données de remplissage.

3. - Procédé selon l'une quelconque des revendications 1 ou 2 **caractérisé en ce qu'**on réalise une étape d'acquisition d'image après chaque étape de remplissage.

4. - Procédé selon l'une des revendications 1 à 3 **caractérisé en ce qu'**on réalise une étape de contrôle du remplissage du pilulier après chaque étape d'acquisition d'image du pilulier (23).

5. - Procédé selon l'une des revendications 1 à 4 **caractérisé en ce qu'**on place une pluralité de pilules (41) dans au moins un logement (40) du pilulier (23), et **en ce que** toutes les pilules placées dans un même logement sont identiques.

6. - Procédé selon l'une des revendications 1 à 5 **caractérisé en ce que** dans au moins une étape de remplissage on place une pluralité de pilules (41) dans au moins un logement (40) du pilulier (23), et **en ce que** les pilules de ladite pluralité de pilules (41) placées dans le pilulier lors d'une même étape de remplissage sont identiques.

7. - Procédé selon l'une des revendications 1 à 6 **caractérisé en ce que** l'étape (30) initiale comprend une étape d'acquisition d'une image, dite image initiale, du pilulier (23) par le dispositif (24) d'acquisition d'images après avoir placé le pilulier (23) en regard du dispositif (24) d'acquisition d'images.

8. - Procédé selon l'une des revendications 1 à 7 **caractérisé en ce qu'**à chaque étape (34) de contrôle la position desdites pilules (41) identifiées est déterminée à partir de moyens de traitement et d'analyse d'image et est comparée à une position indiquée par les données de remplissage de l'étape de remplissage précédant cette étape (34) de contrôle.

9. - Procédé selon l'une des revendications 1 à 8 **caractérisé en ce qu'**à chaque étape (34) de contrôle, une quantité desdites pilules (41) identifiées dans chaque logement (40) est déterminée à partir de moyens de traitement et d'analyse d'images et comparée à une quantité indiquée par les données de remplissage de l'étape de remplissage précédant cette étape (34) de contrôle.

10. - Procédé selon l'une des revendications 1 à 9 **caractérisé en ce qu'**à chaque étape (34) de contrôle, l'image acquise lors de ladite étape d'acquisition courante est comparée à l'image acquise lors de l'étape d'acquisition précédant ladite étape d'acquisition courante uniquement sur certaines zones, dites zones (43) d'intérêt, correspondant à la position des logements (40) du pilulier (23) dont les coordonnés spatiales ont été enregistrées.

11. - Procédé selon l'une des revendications 1 à 10 caractérisé qu'à chaque étape (34) de contrôle, une indication de conformité du remplissage de chacun des logements (40) du pilulier (23) est affichée sur au moins un écran, dit écran de contrôle, après que la conformité du remplissage ait été évaluée.

12. - Procédé selon l'une des revendications 1 à 11 **caractérisé en ce qu'**à chaque étape de remplissage, une indication, dite indication de remplissage, relative aux données de remplissage pour cette étape de remplissage est affichée sur un écran, dit écran (27) d'aide au remplissage.

13. - Dispositif de préparation d'un pilulier (23) comprenant une pluralité de logements (40) de réception d'au moins un médicament, dit pilule (41), sous une forme galénique solide divisée,
dispositif de préparation comprenant un dispositif (24) d'acquisition d'images adapté pour :
- être placé en regard des logements (40) du pilulier (23) lors d'au moins deux étapes de remplissage dans lesquelles on place à chaque étape de remplissage un ensemble d'au moins une pilule (41) dans au moins un logement (40) du pilulier (23) selon des données, dite données de remplissage, d'une prescription médicale pour un traitement thérapeutique d'un patient,
- effectuer au moins deux acquisitions d'image du pilulier (23) par le dispositif (24) d'acquisition d'images dont :
∘ une acquisition d'une image du pilulier (23) après une étape de remplissage,
∘ une autre acquisition d'une image du pilulier (23) après une autre étape de remplissage,
**caractérisé en ce qu'**il comprend un dispositif (25) de traitement de données comprenant des moyens de mettre en oeuvre, après au moins une étape d'acquisition, dite étape d'acquisition courante, une étape (34) de contrôle du remplissage du pilulier (23) dans laquelle :
∘ une image acquise lors de ladite étape d'acquisition courante est comparée à une image acquise lors d'une étape d'acquisition précédant ladite étape d'acquisition courante de façon à identifier les pilules (41) placées entre cette étape d'acquisition précédant ladite étape d'acquisition courante et l'étape d'acquisition courante,
∘ puis une conformité du remplissage est évaluée à partir d'une comparaison entre les pilules (41) identifiées et les données de remplissage.

14. - Programme d'ordinateur comprenant des instructions de code de programme pour l'exécution des étapes d'un procédé selon l'une des revendications 1 à 12 lorsque ledit programme est exécuté sur un ordinateur.

15. - Support d'enregistrement lisible par ordinateur comprenant des instructions de code de programme pour l'exécution des étapes d'un procédé selon l'une des revendications 1 à 12 lorsqu'elles sont exécutées sur un ordinateur.

## Patentansprüche

1. Vorbereitungsverfahren einer Pillenbox (23), umfassend eine Vielzahl von Fächern (40) zur Aufnahme von mindestens einem Medikament, bezeichnet als Pille (41), in einer geteilten festen galenischen Form,
wobei bei dem Verfahren Folgendes durchgeführt wird:
- einen Schritt, bezeichnet als Anfangsschritt (30), in dem die Fächer (40) der Pillenbox (23) gegenüber einer Vorrichtung (24) zur Bilderfassung anordnet werden,
- mindestens zwei Füllschritte, bei denen gemäß den Daten, bezeichnet als Fülldaten, einer ärztlichen Verschreibung für eine therapeutische Behandlung eines Patienten bei jedem Füllschritt eine Einheit von mindestens einer Pille (41) in mindestens ein Fach (40) der Pillenbox (23) platziert wird,
- mindestens zwei Schritte (35) zur Bilderfassung der Pillenbox (23) durch die Bilderfassungsvorrichtung (24) davon:
∘ einen Schritt zum Erfassen eines Bilds der Pillenbox (23) nach einem Füllschritt,
∘ einen weiteren Schritt zum Erfassen eines Bilds der Pillenbox (23) nach einem weiteren Füllschritt,
**dadurch gekennzeichnet, dass** nach mindestens einem Erfassungsschritt, bezeichnet als laufender Erfassungsschritt, ein computerimplementierter Schritt (34) zur Kontrolle der Füllung der Pillenbox (23) durchgeführt wird, wobei:
∘ ein Bild, das bei dem aktuellen Erfassungsschritt erfasst wird, mit einem Bild verglichen wird, das bei einem Erfassungsschritt erfasst wird, der dem aktuellen Erfassungsschritt vorausgeht, um die Pillen (41) zu identifizieren, die zwischen diesem Erfassungsschritt, der dem aktuellen Erfassungsschritt vorausgeht, und dem aktuellen Erfassungsschritt platziert werden,
∘ dann wird eine Konformität der Füllung anhand eines Vergleichs zwischen den identifizierten Pillen (41) und den Fülldaten beurteilt.

2. Verfahren nach Anspruch 1, **dadurch gekennzeichnet, dass** bei dem Schritt (34) einer Kontrolle der Füllung der Pillenbox:
∘ das in dem aktuellen Erfassungsschritt erfasste Bild mit einem Bild verglichen wird, das in dem Erfassungsschritt erfasst wurde, der dem aktuellen Erfassungsschritt chronologisch vorausgeht, um die Pillen (41) zu identifizieren, die zwischen diesem Erfassungsschritt, der dem aktuellen Erfassungsschritt chronologisch vorausgeht, und dem aktuellen Erfassungsschritt platziert werden,
∘ dann wird eine Konformität der Füllung anhand eines Vergleichs zwischen den identifizierten Pillen (41) und den Fülldaten beurteilt.

3. Verfahren nach einem der Ansprüche 1 oder 2, **dadurch gekennzeichnet, dass** nach jedem Füllschritt ein Bildaufnahmeschritt ausgeführt wird.

4. Verfahren nach einem der Ansprüche 1 bis 3, **dadurch gekennzeichnet, dass** nach jedem Schritt der Bilderfassung des Pillenbox (23) ein Schritt zur Kontrolle der Befüllung des Pillenbox ausgeführt wird.

5. Verfahren nach einem der Ansprüche 1 bis 4, **dadurch gekennzeichnet, dass** eine Vielzahl von Pillen (41) in mindestens ein Fach (40) der Pillenbox (23) platziert wird und dass alle Pillen, die in einem gleichen Fach platziert werden, identisch sind.

6. Verfahren nach einem der Ansprüche 1 bis 5, **dadurch gekennzeichnet, dass** in mindestens einem Füllschritt eine Vielzahl von Pillen (41) in mindestens ein Fach (40) der Pillenbox (23) platziert wird, und dass die Pillen der Vielzahl von Pillen (41), die in einem gleichen Füllschritt in der Pillenbox platziert werden, identisch sind.

7. Verfahren nach einem der Ansprüche 1 bis 6, **dadurch gekennzeichnet, dass** der Anfangsschritt (30) einen Schritt zur Erfassung eines Bilds, bezeichnet als Anfangsbild, der Pillenbox (23) durch die Bilderfassungsvorrichtung (24) umfasst, nachdem die Pillenbox (23) gegenüber der Bilderfassungsvorrichtung (24) platziert wurde.

8. Verfahren nach einem der Ansprüche 1 bis 7, **dadurch gekennzeichnet, dass** bei jedem Kontrollschritt (34) die Position der identifizierten Pillen (41) anhand von Bildverarbeitungs- und -analyseeinrichtungen bestimmt und mit einer Position verglichen wird, die durch die Fülldaten des diesem Kontrollschritt (34) vorausgehenden Füllschritts angegeben wird.

9. Verfahren nach einem der Ansprüche 1 bis 8, **dadurch gekennzeichnet, dass** bei jedem Kontrollschritt (34) eine Menge der identifizierten Pillen (41) in jedem Fach (40) anhand von Bildverarbeitungs- und -analyseeinrichtungen bestimmt und mit einer Menge verglichen wird, die durch die Fülldaten des diesem Kontrollschritt (34) vorausgehenden Füllschritts angegeben wird.

10. Verfahren nach einem der Ansprüche 1 bis 9, **dadurch gekennzeichnet, dass** bei jedem Kontrollschritt (34) das bei dem laufenden Erfassungsschritt erfasste Bild mit dem Bild verglichen wird, das bei dem laufenden Erfassungsschritt vorausgehenden Erfassungsschritt erfasst wurde, und zwar nur in bestimmten Zonen, den sogenannten Zonen (43) von Interesse, die der Position der Fächer (40) der Pillenbox (23) entsprechen, deren Raumkoordinaten aufgezeichnet wurden.

11. Verfahren nach einem der Ansprüche 1 bis 10, **dadurch gekennzeichnet, dass** bei jedem Kontrollschritt (34) eine Anzeige der Konformität der Füllung jeder der Fächer (40) der Pillenbox (23) auf mindestens einem Bildschirm, dem sogenannten Kontrollbildschirm, angezeigt wird, nachdem die Konformität der Füllung bewertet wurde.

12. Verfahren nach einem der Ansprüche 1 bis 11, **dadurch gekennzeichnet, dass** bei jedem Füllschritt eine Angabe, bezeichnet als Füllangabe, bezüglich der Fülldaten für diesen Füllschritt auf einem Bildschirm, bezeichnet als Bildschirm (27) zur Füllhilfe, angezeigt wird.

13. Vorbereitungsvorrichtung einer Pillenbox (23), umfassend eine Vielzahl von Fächern (40) zur Aufnahme von mindestens einem Medikament, bezeichnet als Pille (41), in einer geteilten festen galenischen Form,
die Vorbereitungsvorrichtung umfassend eine Vorrichtung (24) zur Bilderfassung, die zu Folgendem angepasst ist:
- bei mindestens zwei Füllschritten gegenüber den Fächern (40) des Pillenbox (23) platziert zu werden, wobei bei jedem Füllschritt gemäß den Daten, bezeichnet als Fülldaten, einer ärztlichen Verschreibung für eine therapeutische Behandlung eines Patienten bei jedem Füllschritt eine Einheit von mindestens einer Pille (41) in mindestens ein Fach (40) der Pillenbox (23) platziert wird,
- Durchführen von mindestens zwei Bilderfassungen der Pillenbox (23) durch die Bilderfassungsvorrichtung (24), davon:
∘ eine Erfassung eines Bilds der Pillenbox (23) nach einem Füllschritt,
∘ eine weitere Erfassung eines Bilds der Pillenbox (23) nach einem weiteren Füllschritt,
**dadurch gekennzeichnet, dass** sie eine Datenverarbeitungsvorrichtung (25) umfasst, umfassend Einrichtungen, um nach mindestens einem Erfassungsschritt, bezeichnet als laufender Erfassungsschritt, einen Schritt (34) zur Kontrolle der Füllung der Pillenbox (23) durchzuführen, wobei:
∘ ein Bild, das bei dem aktuellen Erfassungsschritt erfasst wird, mit einem Bild verglichen wird, das bei einem Erfassungsschritt erfasst wird, der dem aktuellen Erfassungsschritt vorausgeht, um die Pillen (41) zu identifizieren, die zwischen diesem Erfassungsschritt, der dem aktuellen Erfassungsschritt vorausgeht, und dem aktuellen Erfassungsschritt platziert werden,
∘ dann wird eine Konformität der Füllung anhand eines Vergleichs zwischen den identifizierten Pillen (41) und den Fülldaten beurteilt.

14. Computerprogramm, umfassend Programmcodeanweisungen zur Ausführung der Schritte eines Verfahrens nach einem der Ansprüche 1 bis 12, wenn das Programm auf einem Computer ausgeführt wird.

15. Computerlesbares Aufzeichnungsmedium, umfassend Programmcodeanweisungen zur Ausführung von Schritten eines Verfahrens nach einem der Ansprüche 1 bis 12, wenn diese auf einem Computer ausgeführt werden.

## Claims

1. - A method for preparing a pillbox (23) comprising a plurality of cavities (40) for receiving at least one medication, called pill (41), in divided solid galenic form,
wherein the following steps are carried out:
- a step, called initial step (30), wherein the cavities (40) of the pillbox (23) are placed facing an image acquisition device (24),
- at least two filling steps wherein, in each filling step, a group of at least one pill (41) is placed in at least one cavity (40) of the pillbox (23) according to data, called filling data, of a medical prescription for a therapeutic treatment of a patient,
- at least two steps (35) of image acquisition of the pillbox (23) by the image acquisition device (24), including:
∘ a step of acquiring an image of the pillbox (23) after a filling step,
∘ another step of acquiring an image of the pillbox (23) after another filling step,
**characterized in that**, after at least one acquisition step, called current acquisition step, a computer-implemented step (34) of checking the filling of the pillbox (23) is performed, wherein:
∘ an image acquired during said current acquisition step is compared with an image acquired during an acquisition step preceding said current acquisition step so as to identify the pills (41) placed between this acquisition step preceding said current acquisition step and the current acquisition step,
∘ then conformity of the filling is evaluated from a comparison between the pills (41) identified and the filling data.

2. - The method according to claim 1, **characterized in that** during said step (34) of checking the filling of the pillbox:
∘ the image acquired during said current acquisition step is compared with an image acquired during the acquisition step which chronologically precedes said current acquisition step so as to identify the pills (41) placed between this acquisition step which chronologically precedes said current acquisition step and the current acquisition step,
∘ then conformity of the filling is evaluated from a comparison between the identified pills (41) and the filling data.

3. - The method according to any one of claims 1 or 2, **characterized in that** a step of image acquisition is performed after each filling step.

4. - The method according to one of claims 1 to 3, **characterized in that** a step of checking the filling of the pillbox is performed after each step of image acquisition of the pillbox (23).

5. - The method according to one of claims 1 to 4, **characterized in that** a plurality of pills (41) are placed in at least one cavity (40) of the pillbox (23), and **in that** all the pills placed in the same cavity are identical.

6. - The method according to one of claims 1 to 5, **characterized in that**, in at least one filling step, a plurality of pills (41) are placed in at least one cavity (40) of the pillbox (23), and **in that** the pills of said plurality of pills (41) placed in the pillbox during the same filling step are identical.

7. - The method according to one of claims 1 to 6, **characterized in that** the initial step (30) comprises a step of acquiring an image, called initial image, of the pillbox (23) by the image acquisition device (24) after having placed the pillbox (23) facing the image acquisition device (24).

8. - The method according to one of claims 1 to 7, **characterized in that**, in each checking step (34), the position of said identified pills (41) is determined using image processing and analyzing means and is compared with a position indicated by the filling data of the filling step preceding this checking step (34).

9. - The method according to one of claims 1 to 8, **characterized in that**, in each checking step (34), a quantity of said identified pills (41) in each cavity (40) is determined using image processing and analyzing means and is compared with a quantity indicated by the filling data of the filling step preceding this checking step (34).

10. - The method according to one of claims 1 to 9, **characterized in that**, in each checking step (34), the image acquired during said current acquisition step is compared with the image acquired during the acquisition step preceding said current acquisition step only on certain zones, called zones of interest (43), corresponding to the position of the cavities (40) of the pillbox (23) for which the spatial coordinates have been recorded.

11. - The method according to one of claims 1 to 10, **characterized in that**, in each checking step (34), an indication of conformity of the filling of each of the cavities (40) of the pillbox (23) is displayed on at least one screen, called checking screen, after the conformity of the filling has been evaluated.

12. - The method according to one of claims 1 to 11, **characterized in that**, in each filling step, an indication, called filling indication, relating to the filling data for this filling step, is displayed on a screen, called filling assistance screen (27).

13. - A device for preparing a pillbox (23) comprising a plurality of cavities (40) for receiving at least one medication, called pill (41), in divided solid galenic form,
which preparation device comprises an image acquisition device (24) adapted to:
- be placed facing the cavities (40) of the pillbox (23) during at least two filling steps wherein, in each filling step, a group of at least one pill (41) is placed in at least one cavity (40) of the pillbox (23) according to data, called filling data, of a medical prescription for a therapeutic treatment of a patient,
- perform at least two image acquisitions of the pillbox (23) by the image acquisition device (24), including:
∘ acquisition of an image of the pillbox (23) after a filling step,
∘ another acquisition of an image of the pillbox (23) after another filling step,
**characterized in that** it comprises a data processing device (25) comprising means for carrying out, after at least one acquisition step, called current acquisition step, a step (34) of checking the filling of the pillbox (23) wherein:
∘ an image acquired during said current acquisition step is compared with an image acquired during an acquisition step preceding said current acquisition step so as to identify the pills (41) placed between this acquisition step preceding said current acquisition step and the current acquisition step,
∘ then conformity of the filling is evaluated from a comparison between the identified pills (41) and the filling data.

14. - A computer program comprising program code instructions for performing the steps of a method according to one of claims 1 to 12 when said program is executed on a computer.

15. - A computer-readable storage medium comprising program code instructions for performing the steps of a method according to one of claims 1 to 12 when they are executed on a computer.
